# FASCICULE DE BREVET EUROPEEN

(11) **EP 1 802 288 B1**
(45) Date de publication et mention de la délivrance du brevet: **03.11.2010**
(21) Numéro de dépôt: 05805590.6
(22) Date de dépôt: 08.09.2005
(51) Int. Cl.: A61K 9/50, A61P 39/06

(54) **MICROPARTICULES POLYMERIQUES BIODEGRADABLES**
BIOLOGISCH ABBAUBARE POLYMERE MIKROPARTIKEL
BIODEGRADABLE POLYMERIC MICROPARTICLES

(30) Priorité: 09.09.2004 FR 0409564
(43) Date de publication de la demande: 04.07.2007
(73) Titulaire: Alain Dreyer Brevets et Developpement, 84470 Chateauneuf de Gadagne (FR)
(72) Inventeur: DREYER, Alain, F-84470 Châteauneuf de Gadagne (FR); LACAN, Dominique, F-34080 Montpellier (FR); YARD, Christian, F-30250 Combas (FR)
(74) Mandataire: Ahner, Francis
(86) Numéro de dépôt international: PCT/FR2005/002235
(87) Numéro de publication internationale: WO 2006/030111

(56) Documents cités:
- FR-A- 2 822 381
- Y. OHYA ET AL.: "Release behaviour of 5-fluorouracil from chitosan-gel microspheres immobilizing 5-fluorouracil derivative coated with polysaccharides and their cell specific recognition" JOURNAL OF MICROENCAPSULATION, vol. 10, no. 1, janvier 1993 (1993-01), pages 1-9, XP000334990 LONDON GB

## Description

La présente invention concerne des microparticules polymériques biodégradables à base d'un noyau ionique comprenant un extrait végétal actif contenant de la superoxyde dismutase, en particulier un extrait protéique de cucumis melo, enrobé par une couche externe de charge opposée. Suivant la charge du noyau et de la couche externe, ces microparticules peuvent être utilisées pour des applications topiques ou orales, dans des formulations cosmétiques, pharmaceutiques ou alimentaires.

Si l'oxygène est indispensable à notre fonctionnement, il génère pourtant des formes réactives oxygénées toxiques qui ont un effet négatif sur notre organisme.

Dans les systèmes biologiques, les formes réactives oxygénées comprennent des radicaux libres comme le radical superoxyde (0₂·⁻), le radical hydroxyle (OH ·), le monoxyde d'azote (NO·) ou des radicaux peroxydes d'origine lipidique (L-OO·). Ces radicaux libres sont des atomes ou molécules dont la configuration électronique est caractérisée par la présence d'un électron non apparié. Cette particularité les rend instables et ils peuvent rapidement, pour se stabiliser, oxyder de nouvelles molécules biologiques comme les acides nucléiques (ADN), les protéines enzymatiques ou les lipides membranaires et particulièrement les acides gras polyinsaturés (AGPI). L'ion superoxyde est le radical libre le plus couramment et le plus abondamment généré dans les cellules. Il représente donc un danger considérable pour l'organisme. Sa toxicité dépend surtout de sa transformation en d'autres réactifs plus agressifs tels que le radical hydroxyle (OH·).

Notre organisme agit en permanence contre la formation de radicaux libres et on peut envisager plusieurs lignes de défense pour détoxifier la cellule.

La première ligne de défense est assurée par une enzyme appelée la superoxyde dismutase (SOD). Il existe trois formes de SOD : une contenant du cuivre et du zinc, c'est la Cu/Zn SOD ; une autre contenant du manganèse (Mn SOD) et enfin une SOD contenant du fer (Fe SOD°). La SOD joue un rôle clé dans la lutte contre les radicaux libres puisqu'elle permet l'élimination de l'ion superoxyde. Les superoxydes dismutases sont des enzymes capables d'induire la dismutation des ions superoxydes, suivant la réaction :

2O₂ + 2H⁺ → H₂O₂ + 02

Cette élimination de l'ion superoxyde conduit toutefois à la formation de peroxyde d'hydrogène (H₂O₂) qui est également toxique pour la cellule. L'action de la SOD doit donc être complétée par une deuxième ligne de défense qui élimine H₂O₂.

La deuxième ligne de défense est assurée par deux enzymes : la catalase et la glutathion péroxydase-selenium dépendante (SeGPx) qui assurent la destruction de H₂O₂.

Dans des conditions biologiques normales, l'organisme humain produit constamment mais en faibles quantités des radicaux libres qui sont immédiatement neutralisés par les systèmes de défense existants. Dans certains cas, si la production de radicaux libres augmente (tabac, stress, pollution, radiations solaires...) et/ou s'il existe un déficit en substances antioxydantes cela conduit à un déséquilibre oxydant/antioxydant. Ce déséquilibre est de plus en plus étroitement corrélé à de nombreuses pathologies et de nombreux déséquilibres. De ce fait, la recherche de substances antioxydantes a fait l'objet d'un intérêt constant aussi bien dans le domaine de la cosmétologie, de l'agro-alimentaire ou de la médecine.

Le brevet FR 2 287 899 a décrit par exemple l'application en cosmétologie des enzymes superoxydes dismutases et en particulier l'utilisation de ces enzymes dans la préparation de compositions cosmétiques pour le soin de la peau et des cheveux. La SOD a donc été utilisée dans des pathologies induites par des radicaux libres, notamment dans le cas d'inflammations chroniques comme par exemple dans le traitement de la maladie de Crohn (Emerit et al., 1991, Free Rad. Res. Comms, 12-13, 563-569) ou encore dans le cas de fibroses radio-induites (Delanian et al. 1994, Radiotherapy and Oncology, 32, 12-20).

Ces superoxydes dismutases sont notamment extraites d'érythrocytes de boeuf (Markovitz, J. Biol. Chem., 234, p. 40, 1959), d'*Escherichia Coli* (Keele et Fridovitch, J. Biol., 245, p. 6176, 1970) et de souches bactériennes marines (brevets FR 2 225 443 et FR 2 240 277).

Néanmoins, pour des raisons liées notamment aux substances infectieuses que peuvent contenir ces matières, on a tendance à l'heure actuelle à remplacer celles-ci par des substances provenant du règne végétal réputées plus saines. Ces enzymes anti-oxydantes sont en effet présentes dans des quantités variables dans certains végétaux.

Ainsi la demande de brevet FR 2 716 884 décrit un extrait protéique de Cucumis melo présentant une activité enzymatique améliorée superoxyde dismutase, leur procédé de préparation et leur utilisation dans des composition pharmaceutiques ou cosmétiques à usage topique externe. Toutefois, un tel extrait protéique présente des difficultés d'utilisation, les enzymes qu'il contient (superoxyde dismutase en particulier) étant inactivées en présence d'agressions extérieures (U.V. en particulier) ou lors de l'absorption dans des formulations à usage oral (suc gastrique). Il convient alors de mettre au point des formulations spécifiques qui assurent cette protection pour optimiser l'action des actifs antioxydants.

Pour augmenter la demi-vie plasmatique des formes libres de SOD (Cu/Zn, Fe ou Mn), différentes formes modifiées, pour l'administration parentérale ont été proposées ; on peut citer les SOD conjuguées au polyéthylène glycol (SOD-PEG), les SOD conjuguées à l'héparine (SOD-Héparine), les SOD conjuguées à l'albumine (SOD-albumine), les polymères ou copolymères de SOD et les SOD liposomales. Toutefois ces différentes SOD ont l'inconvénient majeur d'être très peu absorbées lorsqu'elles sont administrées par voie orale.

La demande de brevet FR 2 729 296 décrit également une composition pharmaceutique particulière bien adaptée à l'administration orale de la superoxyde dismutase comprenant essentiellement en combinaison une superoxyde dismutase et un composé sélectionné dans le groupe constitué par des céramides, des prolamines et des films polymériques à base desdites prolamines. Toutefois une telle composition n'est pas adaptée à une utilisation par voie topique et en outre ne peut être utilisée par des personnes allergiques au gluten.

L'article de Ohya et al (J. Microencapsulation, 1993, Vol 10, n°1, pages 1-9) décrit des microsphères de gel de chitosan enrobées par des polysaccharides et comprenant du 5-fluorouracile mais ne contenant pas de superoxyde dismutase.

La demande de brevet FR2 822 381 décrit des extraits végétaux actifs, en particulier contenant de la superoxyde dismutase et surtout un extrait protéique de cucumis melo, enrobés et/ou microencapsulés dans un agent liposoluble à base de matière grasse et qui peuvent être utiles dans des applications orales ou topiques. Toutefois, les produits obtenus sont difficilement dispersibles dans des phases hydrophiles.

La demande de brevet WO 98/13030 décrit des matrices particulaires dotées d'un noyau hydrophile d'hydrates de carbone ou de polyols ou de polyamines, réticulés et dérivés dans la masse par des taux variables de groupements ioniques, enrobé d'une couche polymérique hydrophile associée par interactions chimiques, par exemple ioniques, sur laquelle est greffée par des liaisons covalentes des molécules ou des polymères de surface, régissant les interactions de la particule avec le milieu extérieur. Ces matrices peuvent contenir des extraits actifs. Toutefois des molécules ou polymères de surfaces sont toujours greffés sur la face externe de la couche externe. Cette couche externe est d'ailleurs utilisée uniquement pour pouvoir greffer ces molécules ou polymères de surface. En effet, il est impossible de les greffer directement sur le noyau déjà chargé par le principe actif car cela pose de nombreux problèmes. Donc, rien n'incite l'homme du métier à enrober le noyau avec une couche externe non greffée.

Or de façon surprenante, les inventeurs de la présente invention ont découvert qu'un tel enrobage permet une meilleure rétention de l'extrait végétal dans les particules et une protection vis à vis du milieu extérieur. Les microparticules sont donc stables dans des formulations cosmétiques, en particulier destinées à une administration par voie topique, notamment contenant des tensioactifs, et en particulier sous forme de gel ou d'émulsions. Elles sont également stables en milieu gastriques et donc peuvent être administrées par voie orale. L'objectif est donc de disposer d'un produit de nature particulaire dont les composants ne sont pas solubilisés ou déstructurés en présence des constituants d'une formulation cosmétique (crème, gel, lotion...) ou dans l'estomac.

La présente invention concerne donc des microparticules biodégradables polymériques comprenant chacune successivement dans l'ordre du coeur vers l'extérieur:
A) un noyau polymérique biodégradable hydrophile cationique ou anionique constitué par une matrice d'amidon réticulée et dérivé chimiquement dans la masse avantageusement d'hydrates de carbones, ladite matrice étant dérivée dans la masse par des groupements cationiques ou anioniques et ladite matrice incorporant un extrait végétal actif (avantageusement un extrait de melon) contenant de la superoxyde dismutase,
B) une couche externe polymérique biodégradable hydrophile ionique de charge opposée au noyau A, ladite couche étant associée par interactions chimiques, avantageusement par interactions ioniques avec le noyau A et des molécules ou polymères de surface n'étant pas greffés par des liaisons covalentes sur la surface externe de ladite couche B.

Par « extrait végétal actif contenant de la superoxyde dismutase », on entend au sens de la présente invention tout extrait obtenu à partir de végétaux, avantageusement de melon, présentant une activité enzymatique superoxyde dismutase. Avantageusement il s'agit d'un extrait protéique de cucumis melo. De façon encore plus avantageuse le cucumis melo est un descendant de la lignée cellulaire 95LS444 ou de l'une des lignées hybrides issues de 95LS444.

Dans un mode de réalisation particulier, l'activité enzymatique superoxyde dismutase de l'extrait végétal est au moins égale à 5 unités enzymatiques par mg d'extrait, avantageusement au moins égale à 50 unités enzymatiques par mg d'extrait.

L'extrait végétal peut être obtenu par des méthodes bien connues de l'homme du métier, en particulier par le procédé décrit dans la demande de brevet FR 2 716 884. Il est également disponible commercialement chez la société BIONOV sous la marque EXTRAMEL®.

Avantageusement, l'extrait végétal selon la présente invention peut en outre présenter une activité enzymatique catalase. Il peut également comprendre une coenzyme Q10, des vitamines, de l'acide lipoïque, du glutathion, des éléments minéraux présents dans le végétal, tels que le potassium, le magnésium, le calcium et le sélénium etc.

Avantageusement, les microparticules selon la présente invention comprennent entre 2 et 50 % en poids d'extrait végétal par rapport au poids des microparticules, avantageusement 23% en poids d'extrait végétal par rapport au poids des microparticules ou 16 unités SOD par mg de microparticules.

Dans un mode de réalisation particulier, les microparticules selon la présente invention ne comportent pas de couche intermédiaire entre le noyau et la couche externe. Avantageusement les microparticules selon la présente invention sont chacune constituées du noyau A et de la couche B.

Aucune molécule ou polymère de surface n'est greffé sur la couche externe B des microparticules selon la présente invention. En particulier, les microparticules selon la présente invention ne comportent pas de polymères de surface greffés sur la couche externe choisis dans le groupe constitué par les polycarbophiles, les alginates, les polyacrylates, les polyvinylalcools.

Avantageusement la matrice du noyau A et la couche externe B sont choisis indépendamment l'un de l'autre dans la groupe constitué par les polysaccharides, linéaires ou ramifiés, réticulés et dérivés chimiquement par des fonctions ioniques, par exemple l'amidon et ses dérivés, la cellulose et ses dérivés, le dextrane et les polysaccharides dérivés naturellement par des fonctions ioniques, par exemple le chitosan, les gommes, les acides hyaluroniques, les alginates ou les carraghénanes. Toutefois, la matrice du noyau A et la couche externe B sont de charge opposée de façon à ce que la couche B et le noyau A soient associés par des interactions ioniques. Dans un mode de réalisation particulier, la matrice du noyau A est dérivée par des groupements cationiques et la couche externe B est alors un polysaccharide biodégradable anionique, ou la matrice du noyau A est dérivée par des groupements anioniques et la couche externe B est un polysaccharide biodégradable cationique

La matrice du noyau A est formée d'amidon réticulé et avantageusement dérivé chimiquement dans la masse par des groupements ioniques.

Cette matrice est fabriquée par des procédés bien connus de l'homme du métier.

Avantageusement, la couche externe B est un polysaccharide biodégradable anionique ou cationique. Avantageusement la couche externe B est choisie dans le groupe constitué par la carboxyméthyl cellulose, en particulier de sodium ou de calcium, le chitosan et les gommes. Avantageusement, les gommes sont d'origine végétale ou microbienne. Avantageusement, il s'agit de la gomme de xanthane ou de la gomme arabique.

Dans un mode de réalisation particulier, le noyau A est cationique et la couche B est anionique.

Avantageusement, la matrice du noyau A est de l'amidon réticulé et dérivé chimiquement dans la masse par des groupements cationiques et la couche B est un polysaccharide biodégradable anionique, avantageusement de la carboxymethyl cellullose, en particulier de sodium ou de calcium.

Dans un autre mode de réalisation particulier, le noyau A est anionique et la couche B est cationique. Avantageusement, la matrice du noyau A est de l'amidon réticulé et dérivé chimiquement dans la masse par des groupements anioniques et la couche B est un polysaccharide biodégradable cationique, avantageusement du chitosan, ou une gomme. Avantageusement, les gommes sont d'origine végétale ou microbienne. Avantageusement, il s'agit de la gomme de xanthane ou de la gomme arabique.

Dans un mode de réalisation particulier, les microparticules selon la présente invention se trouvent sous forme sèche ou hydratée.

La dimension moyenne des microparticules selon la présente invention est avantageusement comprise entre 10 et 1000 µm, de façon avantageuse entre 100 et 500 µm.

La présente invention concerne en outre un procédé de préparation des microparticules selon la présente invention qui comprend les étapes suivantes :
a) Préparation de microparticules de la matrice du noyau A
b) Incorporation de l'extrait végétal dans les microparticules obtenues à l'étape (a)
c) Enrobage par la couche externe B des microparticules chargées obtenues à l'étape (b)
d) Récupération des microparticules obtenues à l'étape (c).

Avantageusement, l'étape (a) consiste en la réticulation en émulsion de la matrice du noyau A ou en la préparation de la matrice du noyau A suivi d'un broyage pour former des microparticules. Ainsi les microparticules de la matrice du noyau A peuvent être obtenues par plusieurs procédés. Le premier consiste à broyer mécaniquement le gel obtenu par réticulation en masse de la matrice du noyau A. la seconde technique consiste à réaliser directement la matrice sous forme de microparticules par la technique de réticulation en émulsion de la matrice du noyau A dans un liquide non miscible avec la phase réactionnelle. Ce procédé est bien connu de l'homme du métier et permet d'obtenir des noyaux A de taille comprise entre 100 et 500 µm.

La matrice du noyau A peut être préparée par différentes méthodes bien connues de l'homme du métier. En particulier, il s'agit de d'amidons et leurs dérivés, les la matrice du noyau A ionique est alors obtenue par réticulation et dérivation par des procédés bien connues de l'homme du métier.

Le caractère ionique de la matrice du noyau A est obtenu en utilisant un polymère déjà dérivé par des échangeurs ioniques ou en réalisant le greffage sur des polymères neutres de ligands ioniques biocompatibles et biodégradables, selon des procédés bien connus de l'homme du métier.

La modulation des paramètres physicochimiques des microparticules selon la présente invention, principalement le taux de réticulation (Tr) et le taux de greffage ionique (Tg) de la matrice du noyau A, permet de modifier l'efficacité d'incorporation et ainsi la teneur finale en extrait végétal actif, selon l'application concernée.

Ainsi pour des microparticules dont la matrice du noyau A est constituée par de l'amidon réticulé et dérivé dans la masse par des groupements cationiques et la couche B est de la carboxymethyl cellulose, les teneurs finales en extrait végétal actif en fonction de Tr et Tg sont rassemblés dans le tableau 1 suivant :

**Tableau 1 : teneur en SOD en fonction de Tr et Tg**

| Tr | Tg | Teneur en SOD (Unités / mg) |
|---|---|---|
| 20 % | 100 % | 9 |
| 8 % | 100 % | 14 |
| 8 % | 50 % | 19 |
| 8 % | 6 % | 22 |

| | | |
|---|---|---|
| Tr = taux de réticulation, exprimé en pourcentage de pontages par mole d'unité glucose dans le polysaccharide de la matrice du noyau A. Tg = taux de greffage ionique, exprimé en pourcentage de groupements ioniques greffés par unité glucose dans le polysaccharide de la matrice du noyau A. | | |

L'incorporation résulte d'un équilibre entre la densité du maillage interne et la quantité de groupements ioniques greffés interagissant avec l'extrait végétal.

Compte tenu des propriétés des microparticules selon la présente invention, le profil de libération de l'extrait végétal actif peut être modulé selon les applications souhaitées et le type de microparticules fabriquées. Il dépend en effet :
- de la vitesse de dégradation de la microparticule selon la présente invention, déterminée par le taux et le type de réticulation, et de greffage ionique de la matrice du noyau A et le taux et le type d'enrobage polymérique par la couche externe B.
- de la cinétique de désorption de l'extrait végétal actif, liée à son affinité pour la microparticule selon la présente invention.

Pour les administrations topiques, la désorption de l'extrait végétal actif sera accélérée par leur libération physique du fait de l'écrasement des microparticules sur la peau lors de l'application de la crème ou du gel.

Un des intérêts des microparticules selon la présente invention est de permettre l'incorporation d'extrait végétal actif au coeur de la matrice du noyau A tout en disposant de propriétés de surface différentes de celles du de la matrice du noyau A, grâce à la présence de la couche externe B.

Il a ainsi été découvert que, de façon surprenante, les microparticules selon la présente invention présentant des charges de surface positives, apportées par la couche externe B qui est un polymère cationique (chitosan par exemple), ont une affinité très élevée pour la muqueuse intestinale et présentent un caractère bio adhésif particulièrement intéressant pour un ciblage mucosal.

Par ailleurs, la nature covalente des liens assurant la structure tridimensionnelle des microparticules selon la présente invention permet d'assurer une excellente stabilité dans les milieux de dispersion susceptibles d'être utilisés pour les applications concernées. Ainsi, les microparticules selon la présente invention ne sont pas déstructurées ou solubilisées en présence des constituants de formulations cosmétiques ou pharmaceutiques en particulier destinées à une administration par voie topique (crèmes, lotions, gels, etc). La structure des microparticules selon la présente invention n'est en effet pas modifiée en présence de tensioactifs ou de sels.

Ainsi, la formulation de 2 g de microparticules chargées à 16 unités SOD par mg dans une crème cosmétique est stable après 3 mois à température ambiante.

La présente invention concerne également une composition pharmaceutique ou cosmétique comprenant des microparticules selon l'invention et, avantageusement un excipient approprié, avantageusement destinée à un usage orale ou topique pour une administration humaine ou animale. Ces compositions peuvent être formulées pour l'administration aux mammifères, y compris l'homme. La posologie varie selon le traitement et selon l'affection en cause.

Dans un mode de réalisation particulier, la composition pharmaceutique ou cosmétique selon la présente invention peut être formulée pour une administration par voie orale. Avantageusement dans ce cas il s'agit de microparticules selon la présente invention dont le noyau A est anionique et la couche B est cationique. Dans un mode de réalisation avantageux, la matrice du noyau A est formée d'amidon réticulé et dérivé chimiquement dans la masse par des groupements anioniques.

Pour une administration par voie orale, l'ingrédient actif peut être administré sous formes unitaires d'administration, en mélange avec des supports pharmaceutiques classiques, aux animaux ou aux êtres humains. Les formes unitaires d'administrations appropriées comprennent les formes telles que les comprimés, les gélules, les poudres, les granules et les solutions ou suspensions orales.

Avantageusement il s'agit de compléments alimentaires.

Dans un autre mode de réalisation particulier de l'invention, la composition pharmaceutique ou cosmétique peut également être formulée pour une administration par voie topique. Avantageusement dans ce cas il s'agit de microparticules selon la présente invention dont le noyau A est cationique et la couche B est anionique. Dans un mode de réalisation avantageux, la matrice du noyau A est formée d'amidon réticulé et dérivé chimiquement dans la masse par des groupements cationiques.

Dans un mode de réalisation avantageux, la couche B est un polysaccharide biodégradable anionique, avantageusement de la carboxyméthyl cellulose, en particulier de sodium ou de calcium.

Les compositions pharmaceutiques ou cosmétiques destinées à une administration topique selon la présente invention pourront se présenter sous les formes qui sont habituellement connues pour ce type d'administration, c'est à dire notamment les lotions, les mousses, les gels, les dispersions, les sprays, les shampooings, les sérums, les masques, les laits corporels ou les crèmes par exemple, avec des excipients permettant notamment une pénétration cutanée afin d'améliorer les propriétés et l'accessibilité du principe actif. Ces compositions contiennent généralement outre la composition selon la présente invention, un milieu physiologiquement acceptable, en général à base d'eau ou de solvant, par exemple des alcools, des éthers ou des glycols. Elles peuvent également contenir des agents tensioactifs, des conservateurs (type paraben), des agents stabilisants, des émulsifiants, des épaississants, d'autres principes actifs conduisant à un effet complémentaire ou éventuellement synergique, des oligo-éléments, des huiles ou des huiles essentielles, des parfums, des colorants, du collagène, des filtres chimiques ou minéraux, des agents hydratants ou des eaux thermales, des acrylates, des carbomères (type carbopol®), des émollients, des gélifiants, des agents antioxydants, tels que le BHT ou BHA, des silicones.

La présente invention concerne également des microparticules selon la présente invention pour leur utilisation en tant que médicament, avantageusement destiné à un usage topique ou orale

Avantageusement, elle concerne des microparticules selon la présente invention pour leur utilisation en tant que médicament destiné à traiter ou à prévenir les allergies, l'eczéma, le vitiligo, le psoriasis, le lupus, la fibrose cutanée, à éliminer les mélanomes, à améliorer les cicatrisations des greffes, à prévenir les allergies ou à protéger l'épiderme contre les radiations U.V et contre les allergies, à traiter l'asthme, l'anémie, la stérilité mâle, les maladies inflammatoires telles que l'anthropathie ou les maladies dégénératives dues à des dégénérescences cellulaires et/ou organiques, en particulier choisies dans le groupe constitué par la maladie de Crohn, la maladie de Parkinson, la maladie d'Alzheimer, les cancers colorectaux, les fibroses d'origine variées, les dégénérescences dues à un agent infectieux telles que le SIDA ou l'hépatite C ou les dégénérescences liées à l'utilisation d'un médicament.

La présente invention concerne également l'utilisation de microparticules selon la présente invention pour la fabrication d'un médicament à usage topique destiné à traiter les allergies, l'eczéma, le vitiligo, le psoriasis, le lupus, la fibrose cutanée à éliminer les mélanomes, à améliorer les cicatrisations des greffes, à prévenir les allergies ou à protéger l'épiderme vis-à-vis des radiations U.V.

La présente invention concerne également l'utilisation de microparticules selon la présente invention pour la fabrication d'un médicament à usage orale destiné à protéger l'épiderme vis-à-vis des radiations U.V., à traiter ou prévenir les allergies, à traiter l'asthme, l'anémie, la stérilité mâle, les maladies inflammatoires telles que l'anthropathie ou les maladies dégénératives dues à des dégénérescences cellulaires et/ou organiques, en particulier choisies dans le groupe constitué par la maladie de Crohn, la maladie de Parkinson, la maladie d'Alzheimer, les cancers colorectaux, les fibroses d'origine variées, les dégénérescences dues à un agent infectieux telles que le SIDA ou l'hépatite C ou les dégénérescences liées à l'utilisation d'un médicament.

La présente invention concerne également l'utilisation d'un complément alimentaire contenant des microparticules selon la présente invention, avantageusement en tant qu'apport énergétique, apport dynamisant, ou aide à la récupération et à la remise en forme.

Les exemples suivants sont donnés à titre indicatif, non limitatif.

### Exemple 1 : Préparation de microparticules de la matrice du noyau A polysaccharidique réticulée cationique (T_{R} 1/15 - T_{G} 1/20) des microparticules selon la présente invention

Dans un réacteur de 2,5 litres, 100 g d'amidon ayant un poids moléculaire d'environ 10 000 (commercialisé par la société Roquette) sont dispersés dans 300 ml d'eau contenant 1 gramme de borohydrure de sodium. Après 2 heures d'agitation, on ajoute 100 ml de soude 4 N. Lorsque la solution est homogène, on ajoute 4,1 ml de chlorure de (2,3)époxypropyl)triméthylammonium en solution aqueuse à 75%. Après 2 heures d'agitation, on ajoute 3,2 ml d'épichlorhydrine en maintenant l'agitation pendant 4 heures supplémentaires. La solution est alors laissée au repos pendant 40 heures : on obtient un gel translucide et cassant. Ce gel est repris dans 2 litres d'eau, le pH est ajusté à 5 par addition d'HCl 2 N puis le gel est lavé 4 fois dans 5 litres d'eau distillée. Il est ensuite broyé à l'aide d'une turbine Ultraturrax pendant 5 minutes à 2000 t/min. Les microparticules obtenues ont un diamètre compris entre 100 et 500 microns.

### Exemple 2: Préparation de microparticules de la matrice du noyau A polysaccharidique réticulée (Tr = 20%) anionique des microparticules selon la présente invention

Dans un réacteur de 800 ml, 20 g de trimétaphosphate de sodium sont dissous dans 180 ml d'eau osmosée sous agitation. 100 g de d'amidon (commercialisé par la société Roquette) ayant un poids moléculaire d'environ 10 000 sont ajoutés à la solution obtenue et agitée jusqu'à dissolution complète à température ambiante pendant environ 2 heures. Une solution de carbonate de sodium (16 g dans 60 ml d'eau osmosée) est préparée puis ajoutée à la solution d'amidon et de trimétaphosphate de sodium sous agitation.

La solution obtenue est ensuite placée dans un bain-marie à 50°C pendant 8 min puis dispersée sous forte agitation dans 300 ml de paraffine liquide. Le mélange est laissé sous agitation pendant 20 h à température ambiante. Les microparticules formées (taille entre 100 et 500 µm) sont filtrées, lavées à l'eau osmosée et le pH est ajusté si nécessaire entre 6,5 et 7,5.

### Exemple 3 : Préparation d'un extrait végétal actif (décrit dans la demande de brevet FR2 716 884)

5g de pulpe d'un cucumis melo hybride issu de la lignée cellulaire 95LS444 (décrite dans la demande internationale WO 92/02622) sont broyés dans un mortier à froid. Un volume tampon Phosphate 50 mM (pH :7,5 ; 5EDTA 1 mM ; glycérol 5 %) équivalent à 3 fois la masse végétal est ajouté. Après homogénéisation, la suspension est centrifugée à 5000 g à 4°C pendant 30 minutes. Le surnageant est ensuite récupéré et filtré. Cet extrait brut sert à la détermination de l'activité superoxyde dismutase. Elle est mesurée à l'aide d'un kit de dosage SOD 525 fourni par la société Bioxytech. Cette méthode donne des résultats comparable à ceux obtenus par la méthode de McCord et Fridovitch (inhibition par la SOD de la réduction du cytochrome C par la xanthine-xanthine oxydase).

### Exemple 4 : Préparation de microparticules de noyau A chargées en extrait végétal actif à partir de microparticules sous forme sèche selon l'exemple 1.

Dans un réacteur de 250 ml, 20 ml d'extrait végétal actif obtenu selon l'exemple 3 titré à 3,5 unités SOD par litre sont ajoutés à 3,5 g de microparticules sous forme sèche obtenues après lyophilisation des microparticules selon l'exemple 1, tout en assurant une agitation lente. Après 30 minutes d'agitation lente, les microparticules sont laissées au repos puis récupérées.

### Exemple 5 : Préparation de microparticules de noyau A chargées en extrait végétal actif à partir de microparticules sous forme hydratée selon l'exemple 1.

Dans un réacteur de 250 ml, 20 ml d'extrait végétal obtenu selon l'exemple 3 titré à 3,5 unités SOD par litre sont ajoutés à 200 g de microparticules sous forme hydratée obtenues selon l'exemple 1 (Taux de matière sèche = 1,7 %) tout en assurant une agitation lente. Après 30 minutes d'agitation lente, les microparticules sont laissées au repos pendant 30 minutes puis récupérées.

### Exemple 6 : Préparation de microparticules de noyau A chargées en extrait végétal actif à partir de microparticules sous forme hydratée selon l'exemple 2.

Dans un réacteur de 250 ml, 20 ml d'extrait végétal obtenu selon l'exemple 3 titré à 3,5 unités SOD par litre sont ajoutés à 200 g de microparticules sous forme hydratée obtenues selon l'exemple 2 (Taux de matière sèche = 1,3 %) tout en assurant une agitation lente. Après 30 minutes d'agitation lente, les microparticules sont laissées au repos pendant 30 minutes puis récupérées.

### Exemple 7 : Enrobage polymérique des microparticules chargées en extrait végétal obtenues selon les exemples 4 ou 5

1 g de microparticules chargées en extrait végétal obtenues selon l'exemple 4 ou 5 est placé sous agitation lente et additionné d'une solution de carboxyméthylcellulose à 20 g/l. Après 30 minutes d'agitation, les microparticules sont lavées par tamisage puis immédiatement congelées et lyophilisées.

Les microparticules obtenues contiennent 23 % d'extrait végétal actif (16 unités SOD par mg).

### Exemple 8 : Enrobage polymérique des microparticules chargées en extrait végétal obtenues selon l'exemple 6

1 g de microparticules chargées en extrait végétal obtenues selon l'exemple 6 est placé sous agitation lente et additionné d'une solution de chitosan à 10 g par 1 d'acide acétique dilué (0,3 M). Après 30 minutes d'agitation, les microparticules sont lavées par tamisage puis immédiatement congelées et lyophilisées.

Les microparticules obtenues contiennent 23 % d'extrait végétal actif (16 unités SOD par mg).

### Exemple 9 : Réalisation d'une préparation pour application topique contenant des microparticules selon la présente invention.

La préparation est un gel réalisé de façon bien connue de l'homme du métier en utilisant les ingrédients et proportions indiqués dans le tableau 2 ci après.

**Tableau 2 : composition de la préparation pour application topique**

| INGREDIENTS | % en poids |
|---|---|
| Microparticules selon l'exemple 7 | 2 % |
| Sepigel 305 (Seppic) | 2% |
| Huile de Macadamia (Soetenaey) | 5 % |
| Conservateur (Sepicide® - SEPPIC) | 0,5 % |
| Ethoxydiglycol (Transcutol® - Gattefossé) | 0,5 % |
| Eau | qsp 100 % |

Le pH dans le gel est ramené à pH = 7,2 par ajout de NaOH 10N. On obtient un gel translucide, ayant une teneur en enzyme active de 320 unités SOD par g.

### Exemple 10 : Réalisation d'une formulation pour la voie orale contenant des microparticules selon la présente invention

La préparation est un comprimé réalisé de façon bien connue de l'homme du métier en utilisant les ingrédients et proportions indiqués ci après.

Pour un comprimé de 500 mg dosé à environ 250 U SOD par comprimé

| Ingrédients | quantité en mg |
|---|---|
| Microparticules selon l'exemple 8 | 16 |
| Gluconate de zinc | 56 |
| Cellulose microcristalline | 200 |
| Phosphate de Calcium | 200 |
| Enrobage qs | 28 |
| Total | 500 |

### Exemple 11 : Capacité et stabilité d'incorporation en milieu physiologique des microparticules obtenues selon l'exemple 7.

L'incorporation de l'extrait végétal a été testée sur des matrices polysaccharidiques réticulées cationiques seules (obtenues selon l'exemple 1) ou enrobées d'un polymère anionique, la carboxyméthylcellulose (en utilisant le procédé selon l'exemple 7). Le taux d'incorporation puis la stabilité de l'incorporation, c'est-à-dire la quantité d'extrait végétal actif restant incorporé après incubation en milieu physiologique modèle, ont été mesurés et rassemblés dans les tableaux 3 et 4 suivants.

La quantification de l'extrait végétal actif incorporé a été réalisée par dosage de l'activité superoxyde dismutase selon la méthode de Beauchamp et Fridovich (1971) (in Anal. Biochem. 44 (1971) 276).

**Tableau 3 : Taux d'incorporation (exprimés en unités SOD par mg de microparticules) :**

| | |
|---|---|
| Matrices polysaccharidiques cationiques (MPC) (exemple 1) | 17 u /mg |
| Matrices polysaccharidiques cationiques enrobées de carboxyméthylcellulose (MPC-CMC) (exemple 7) | 16 u / mg |

### Stabilité de l'incorporation en milieu physiologique :

Les microparticules chargées en extrait végétal actif ont été placées dans un tampon phosphate salin à pH 7,4 et la quantité d'enzyme active restant à l'intérieur des particules a été mesurée.

**Tableau 4 : Stabilité de l'incorporation en milieu physiologique**

| | | | | | |
|---|---|---|---|---|---|
| | Activité enzymatique dans les particules après incubation à pH 7,4 (% enzyme active par rapport à la teneur initiale) | | | | |

| | T0 | T0 + 15 min | To+60 min | T0+120min | T0+180 min |
|---|---|---|---|---|---|
| MPC | 100% | 86% | 78% | 75% | 66% |
| MPC-CMC (exemple 7) | 100% | 95% | 95% | 94% | 90% |

L'enrobage des microparticules par un polymère anionique crée un effet de barrière qui limite le relargage de l'extrait végétal actif au cours du temps.

Par rapport à la demande de brevet WO98/13030, l'enrobage des matrices polysaccharidiques du noyau A par la carboxyméthylcellulose (CMC) évite d'avoir recours à une étape complémentaire de synthèse chimique, consistant à dériver de façon covalente un polymère ionique avec des motifs d'intérêt de surface. Il a été montré que la CMC apporte de nombreux avantages, à savoir la création d'un effet de barrière permettant une meilleure rétention de l'extrait végétal actif dans les microparticules selon la présente invention et une protection vis-à-vis du milieu extérieur. Un greffage supplémentaire n'est donc pas nécessaire. Le procédé de fabrication des microparticules selon la présente invention est donc simplifié et les coûts de production en sont nettement diminués.

La nature polymérique réticulée des microparticules selon la présente invention leur confère une stabilité physico-chimique totale dans des formulations cosmétiques ou pharmaceutiques, notamment destinées à une administration par voie topique telles que par exemple les gels et émulsions. Les polysaccharides ainsi réticulés ne sont pas sensibles à une éventuelle déstructuration, en particulier par des tensioactifs, qui ne modifient pas leur structure chimique et leur agencement tridimensionnel.

### Exemple 12 : Capacité et stabilité d'incorporation en milieu gastrique des microparticules selon la présente invention dont le noyau A est anionique et la couche externe B est du chitosan (obtenues selon l'exemple 8).

### Stabilité de l'extrait végétal actif incorporée en milieu gastrique :

L'effet de barrière créé par le polymère de couche externe B enrobant le noyau A apporte également une protection qui a été montrée en incubant les microparticules anioniques enrobées de chitosan (MPA-CHIT) et chargées en extrait végétal actif obtenues selon l'exemple 8 selon la présente invention dans un milieu gastrique modèle (Conditions Pharmacopée Européenne), conditions dans lesquelles l'extrait végétal actif est dénaturé rapidement.

Les résultats sont rassemblés dans le tableau 5 ci-après.

| | Activité enzymatique SOD après incubation en milieu gastrique (% enzyme active par rapport à la teneur initiale) | | | | |
|---|---|---|---|---|---|
| | T0 | T0 + 15 min | T0+30 min | T0+45 min | T0+60mi n |
| Extrait végétal actif non incorporé | 100% | 10 % | 2 % | 0 | 0 |
| MPA-CHIT (exemple 8) | 100% | 85 % | 62 % | 38 % | 34 % |

## Revendications

1. Microparticules biodégradables polymériques de dimension moyenne comprise entre 10 et 1000 µm comprenant chacune successivement dans l'ordre du coeur vers l'extérieur:
A) un noyau polymérique biodégradable hydrophile cationique ou anionique constitué par une matrice d'amidon réticulé et dérivé chimiquement dans la masse par des groupements cationiques ou anioniques et ladite matrice incorporant un extrait végétal actif contenant de la superoxyde dismutase,
B) une couche externe polymérique biodégradable hydrophile ionique de charge opposée au noyau A, ladite couche étant associée par interactions chimiques, avantageusement ioniques avec le noyau A et des molécules ou polymères de surface n'étant pas greffés par des liaisons covalentes sur la surface externe de ladite couche B.

2. Microparticules selon la revendication 1 **caractérisées en ce que** l'extrait végétal est un extrait protéique de cucumis melo.

3. Microparticules selon la revendication 2 **caractérisées en ce que** le cucumis melo est un descendant de la lignée cellulaire 95LS444 ou de l'une des lignées hybrides issues de 95LS444.

4. Microparticules selon l'une quelconque des revendications précédentes **caractérisées en ce qu'**elles sont chacune constituée du noyau A et de la couche B.

5. Microparticules selon l'une quelconque des revendications précédentes **caractérisées en ce qu'**elles se trouvent sous forme sèche ou hydratée.

6. Microparticules selon l'une quelconque des revendications précédentes **caractérisées en ce que** la couche externe B est un polysaccharide biodégradable anionique ou cationique.

7. Microparticules selon l'une quelconque des revendications précédentes **caractérisées en ce qu'**elles comprennent entre 2 et 50 % en poids d'extrait végétal actif par rapport au poids des microparticules, avantageusement 23% en poids d'extrait végétal actif par rapport au poids des microparticules.

8. Microparticules selon l'une quelconque des revendications précédentes **caractérisées en ce que** le noyau A est cationique et la couche B est anionique, avantageusement la matrice du noyau A est de l'amidon réticulé et dérivé chimiquement dans la masse par des groupements cationiques et la couche B est de la carboxyméthyl cellulose.

9. Microparticules selon l'une quelconque des revendications 1 à 7 **caractérisées en ce que** le noyau A est anionique et la couche B est cationique, avantageusement la matrice du noyau A est de l'amidon réticulé et dérivé chimiquement dans la masse par des groupements anioniques et la couche B est du chitosan.

10. Procédé de préparation des microparticules selon l'une quelconque des revendications précédentes **caractérisé en ce qu'**il comprend les étapes suivantes :
a) Préparation de microparticules de la matrice du noyau A ;
b) Incorporation de l'extrait végétal dans les microparticules obtenues à l'étape (a) ;
c) Enrobage par la couche externe B des microparticules chargées obtenues à l'étape (b) ;
d) Récupération des microparticules obtenues à l'étape (c).

11. Procédé selon la revendication 10 **caractérisé en ce que** l'étape (a) consiste en la réticulation en émulsion de la matrice du noyau A ou en la préparation de la matrice du noyau A suivi d'un broyage pour former des microparticules.

12. Composition cosmétique ou pharmaceutique comprenant des microparticules selon l'une quelconque des revendications 1 à 9, avantageusement destinée à un usage topique ou orale.

13. Microparticules selon l'une quelconque des revendications 1 à 9 pour leur utilisation en tant que médicament, avantageusement destiné à un usage topique ou orale.

14. Microparticules selon la revendication 13 pour leur utilisation en tant que médicament destiné à traiter ou à prévenir les allergies, l'eczéma, le vitiligo, le psoriasis, le lupus, la fibrose cutanée, à éliminer les mélanomes, à améliorer les cicatrisations des greffes, à prévenir les allergies ou à protéger l'épiderme contre les radiations U.V et contre les allergies, à traiter l'asthme, l'anémie, la stérilité mâle, les maladies inflammatoires telles que l'anthropathie ou les maladies dégénératives dues à des dégénérescences cellulaires et/ou organiques, en particulier choisies dans le groupe constitué par la maladie de Crohn, la maladie de Parkinson, la maladie d'Alzheimer, les cancers colorectaux, les fibroses d'origine variées, les dégénérescences dues à un agent infectieux telles que le SIDA ou l'hépatite C ou les dégénérescences liées à l'utilisation d'un médicament.

15. Complément alimentaire comprenant des microparticules selon la revendication 9.

16. Utilisation d'un complément alimentaire selon la revendication 15 en tant qu'apport énergétique, apport dynamisant, ou aide à la récupération et à la remise en forme.

## Claims

1. Polymeric, biodegradable microparticles of mean size between 10 and 1000 µm, each successively comprising from the core towards the outside:
A) a cationic or anionic, hydrophilic, biodegradable polymeric nucleus consisting of a cross-linked starch matrix, chemically derived in the mass by cationic or anionic groups, and said matrix incorporating an active plant extract containing superoxide dismutase,
B) an ionic, hydrophilic, biodegradable polymeric outer layer of opposite charge to the nucleus A, said layer being associated by chemical advantageously ionic interactions with the nucleus A, and molecules or surface polymers not being grafted by covalent bonds on the outer surface of said layer B.

2. Microparticles according to claim 1, **characterized in that** the plant extract is a protein extract of *cucumis melo.*

3. Microparticles according to claim 1, **characterized in that** the *cucumis melo* is a descendant of the 95LS444 cell line or of one of the hybrid lines derived from 95LS444.

4. Microparticles according to any of the preceding claims, **characterized in that** they each consist of the nucleus A and of layer B.

5. Microparticles according to any of the preceding claims, **characterized in that** they are in dry or hydrated form.

6. Microparticles according to any of the preceding claims, **characterized in that** the outer layer B is an anionic or cationic biodegradable polysaccharide.

7. Microparticles according to any of the preceding claims, **characterized in that** they contain between 2 and 50 weight % of active plant extract relative to the weight of the microparticles, advantageously 23 weight % of active plant extract relative to the weight of the microparticles.

8. Microparticles according to any of the preceding claims, **characterized in that** the nucleus A is cationic and the layer B is anionic, advantageously the matrix of the nucleus A is cross-linked starch chemically derived in the mass by cationic groups and layer B is carboxymethyl cellulose.

9. Microparticles according to any of claims 1 to 7, **characterized in that** the nucleus A is anionic and layer B is cationic, advantageously the matrix of the nucleus A is cross-linked starch chemically derived in the mass by anionic groups, and layer B is chitosan.

10. Method to prepare microparticles according to any of the preceding claims, **characterized in that** it comprises the following steps:
a) Preparing microparticles of the matrix of nucleus A;
b) Incorporating the plant extract in the microparticles obtained at step (a);
c) Coating the loaded microparticles obtained at step (b) with the outer layer B;
d) Collecting the microparticles obtained at step (c).

11. Method according to claim 10, **characterized in that** step (a) consists of emulsion cross-linking the matrix of nucleus A, or of preparing the matrix of nucleus A followed by grinding to form microparticles.

12. Cosmetic or pharmaceutical composition containing microparticles according to any of claims 1 to 9, advantageously intended for topical or oral use.

13. Microparticles according to any of claims 1 to 9 for their use as medicinal product, advantageously intended for topical or oral use.

14. Microparticles according to claim 13 for their use as medicinal product intended to treat or prevent allergies, eczema, vitiligo, psoriasis, lupus, skin fibrosis, to eliminate melanomas, to improve graft healing, to prevent allergies or to protect the epidermis against UV radiation and against allergies, to treat asthma, anaemia, male infertility, inflammatory diseases such as anthropathy or degenerative diseases due to cell and/or organic degeneration, in particular in the group consisting of Crohn's disease, Parkinson's disease, Alzheimer's disease, colorectal cancers, fibroses of varied origin, degeneration due to an infectious agent such as AIDS or hepatitis C, or degeneration due to the use of a medicinal product.

15. Food supplement containing microparticles according to claim 9.

16. Use of a food supplement according to claim 15 as energy intake, energy booster or aid to recovery and fitness.

## Patentansprüche

1. Biologisch abbaubare Polymer-Mikropartikel mittlerer Größe zwischen 10 und 1000 µm inklusive, von denen jedes nacheinander vom Zentrum nach außen umfasst:
A) einen kationischen oder anionischen hydrophilen biologisch abbaubaren Polymerkern, der von einer Matrix retikulierter und in der Masse chemisch von kationischen oder anionischen Verbindungen abgeleiteter Stärke gebildet wird, wobei die Matrix einen aktiven pflanzlichen Extrakt einschließt, der Superoxid-Dismutase enthält,
B) eine ionische hydrophile biologisch abbaubare äußere, gegenüber dem Kern entgegengesetzt geladene Polymerschicht, wobei die Schicht durch chemische Interaktionen verbunden ist, in vorteilhafter Weise ionischen, mit dem Kern A und Molekülen oder Polymeren der Oberfläche, die nicht durch kovalente Verbindungen auf die externen Oberfläche der Schicht B transplantiert sind.

2. Mikropartikel nach Anspruch 1, **dadurch gekennzeichnet, dass** der pflanzliche Extrakt ein Proteinextrakt aus Cucumis melo ist.

3. Mikropartikel nach Anspruch 2, **dadurch gekennzeichnet, dass** der Cucumis melo ein Abkömmling der Zelllinie 95LS444 oder einer der aus 95LS444 hervorgegangenen Hybridlinien ist.

4. Mikropartikel nach einem der vorangehenden Ansprüche, **dadurch gekennzeichnet, dass** jeder von ihnen aus dem Kern A und der Schicht B besteht.

5. Mikropartikel nach einem der vorangehenden Ansprüche, **dadurch gekennzeichnet, dass** sie in trockener oder feuchter Form vorliegen.

6. Mikropartikel nach einem der vorangehenden Ansprüche, **dadurch gekennzeichnet, dass** die äußere Schicht B ein anionisches oder kationisches biologisch abbaubares Polysaccharid ist.

7. Mikropartikel nach einem der vorangehenden Ansprüche, **dadurch gekennzeichnet, dass** sie im Verhältnis zum Gewicht der Mikropartikel zwischen 2 und 50 Gew.-% aktiven pflanzlichen Extrakt, in vorteilhafter Weise im Verhältnis zum Gewicht der Mikropartikel 23 Gew.-% aktiven pflanzlichen Extrakt, enthalten.

8. Mikropartikel nach einem der vorangehenden Ansprüche, **dadurch gekennzeichnet, dass** der Kern A kationisch ist und die Schicht B anionisch ist, in vorteilhafter Weise die Matrix des Kerns A retikulierte und in der Masse chemisch von kationischen Verbindungen abgeleitete Stärke ist und die Schicht B Carboxylmethylcellulose ist.

9. Mikropartikel nach einem der Ansprüche 1 bis 7, **dadurch gekennzeichnet, dass** der Kern A anionisch ist und die Schicht B kationisch ist, in vorteilhafter Weise die Matrix des Kerns A retikulierte und in der Masse chemisch von anionischen Verbindungen abgeleitete Stärke ist und die Schicht B Chitosam ist.

10. Verfahren zur Herstellung von Mikropartikeln nach einem der vorangehenden Ansprüche, **dadurch gekennzeichnet, dass** es die folgenden Schritte umfasst:
a) Herstellen von Mikropartikeln der Matrix des Kerns A,
b) Einarbeiten des pflanzlichen Extrakts in die in Schritt (a) hergestellten Mikropartikel,
c) Umhüllen der in Schritt (b) hergestellten geladenen Mikropartikel durch die äußere Schicht B,
d) Gewinnen der in Schritt (c) hergestellten Mikropartikel.

11. Verfahren nach Anspruch 10, **dadurch gekennzeichnet, dass** der Schritt (a) in der Retikulierung in Emulsion der Matrix des Kerns A oder in der Herstellung der Matrix von Kern A, gefolgt von einer Zerkleinerung, um Mikropartikel zu bilden, besteht.

12. Kosmetische oder pharmazeutische Zusammensetzung, die Mikropartikel nach einem der Ansprüche 1 bis 9 umfasst, die in vorteilhafter Weise für eine topische oder orale Anwendung bestimmt ist.

13. Mikropartikel nach einem der Ansprüche 1 bis 9 für ihre Anwendung als Arzneimittel, das in vorteilhafter Weise für eine topische oder oralen Anwendung bestimmt ist.

14. Mikropartikel nach Anspruch 13 für ihre Anwendung als Arzneimittel, das dazu bestimmt ist, Allergien, Ekzeme, Vitiligo, Psoriasis, Lupus, Hautfibrose zu behandeln oder vorzubeugen, Melanome zu entfernen, die Heilung von Transplantaten zu verbessern, Allergien vorzubeugen oder die Haut vor UV-Strahlen und Allergien zu schützen, Asthma, Anämie, männliche Sterilität, entzündliche Erkrankungen wie die Anthropatie oder degenerative Erkrankungen zu
behandeln, die auf Zell- und/oder Organdegeneration zurückzuführen sind, die insbesondere aus der Gruppe ausgewählt sind, die gebildet wird von Morbus Crohn, der Parkinson-Krankheit, der Alzheimer-Krankheit, den kolorektalen Krebserkrankungen, den Fibrosen unterschiedlichster Ursache, den degenerativen Erkrankungen, die auf ein infektiöses Mittel zurückzuführen sind, wie AIDS oder Hepatitis C, oder den degenerativen Erkrankungen, die mit der Verwendung eines Arzneimittels verbunden sind.

15. Nahrungsergänzung, die Mikropartikel nach Anspruch 9 umfasst.

16. Verwendung einer Nahrungsergänzung nach Anspruch 15 als Energiezufuhr, dynamisierende Zufuhr oder Hilfe für die Erholung oder Wiedererlangung von Fitness.
